# FASCICULE DE BREVET EUROPEEN

(11) **EP 0 486 395 B1**
(45) Date de publication et mention de la délivrance du brevet: **09.11.1994**
(21) Numéro de dépôt: 91403059.8
(22) Date de dépôt: 14.11.1991
(51) Int. Cl.: A61K 9/18, A61K 31/07, A61K 31/20, A61K 47/32, A61K 47/48, A61K 7/48

(54) **Gel aqueux à base d'acide rétinoique et d'hydroxypropyl-bêta-cyclodextrine**
Wässriges Gel von Retinsäure und Hydroxypropyl-beta-Zyklodextrin
Aqueous gel based on retinoic acid and hydroxypropyl-beta-cycclodextrin

(30) Priorité: 15.11.1990 LU 87843
(43) Date de publication de la demande: 20.05.1992
(73) Titulaire: CENTRE INTERNATIONAL DE RECHERCHES DERMATOLOGIQUES GALDERMA - CIRD GALDERMA, F-06565 Valbonne (FR)
(72) Inventeur: Rolland, Alain, F-06530 Peymeinade (FR); Shroot, Braham, F-06600 Antibes (FR)
(74) Mandataire: Stalla-Bourdillon, Bernard

(56) Documents cités:
- EP-A- 0 149 197
- EP-A- 0 396 184
- WO-A-82/00251
- WO-A-90/14082
- US-A- 4 727 064

## Description

La présente invention a pour objet un gel aqueux contenant, à l'état solubilisé, de l'acide rétinoïque all-trans ou 13-cis et son utilisation en médecine humaine et en cosmétique.

Le nouveau gel selon l'invention trouve tout particulièrement une application dans le traitement des différentes formes d'acné, du psoriasis et autres affections dermatologiques liées à un désordre de la kératinisation (différenciation-prolifération) et certaines affections à composantes inflammatoires et/ou immunoallergiques et dans certaines maladies de dégénérescence du tissu conjonctif. Ce nouveau gel peut également être utilisé pour lutter contre le vieillissement cutané actinique, pour atténuer ou supprimer les taches pigmentaires cutanées et pour favoriser la cicatrisation. Il peut également trouver une application dans le domaine ophtalmologique, notamment pour le traitement des cornéopathies et présente également une activité anti-tumorale.

L'acide rétinoïque ou trétinoïne (DCI) est un agent thérapeutique préconisé dans le traitement local des différentes formes d'acné ainsi que dans le traitement de certains troubles de la kératinisation.

Diverses formes galéniques ont été proposées, notamment des gels, des tampons, des crèmes ainsi que des solutions à des concentrations en acide rétinoïque variant de 0,025% à 0,3%, et dont l'excipient est essentiellement constitué d'alcool éthylique à 95°, en vue d'en obtenir une bonne solubilisation.

Il existe également des médicaments destinés à l'administration orale contenant des rétinoïdes tels que l'acide rétinoïque 13-cis ou l'étrétinate pour le traitement des acnés graves, du psoriasis et autres troubles sérieux de la kératinisation.

L'inconvénient majeur de l'acide rétinoïque est, par voie générale, sa toxicité systémique (tératogénicité et effets indésirables dus à l'hypervitaminose A) et, par voie locale, son action irritante pouvant nécessiter dans certains cas l'arrêt du traitement.

Les solutions alcooliques à 0,2 et 0,3%, très fortement dosées en acide rétinoïque, sont par ailleurs tout à fait contre-indiquées dans le traitement de l'acné et réservées uniquement au traitement des troubles de la kératinisation.

Il a par ailleurs été constaté que la forme "gel dermique" dosée à 0,025% pouvait également présenter un effet irritant, ceci notamment du fait de la nature de l'excipient contenant un fort pourcentage en alcool éthylique à 95°.

Jusqu'à présent, il n'a pas été possible de réaliser des formes galéniques entièrement aqueuses contenant, à l'état solubilisé, l'acide rétinoïque all-trans ou 13-cis et présentant un pH essentiellement neutre et une bonne stabilité dans le temps et ne provoquant pas d'irritation cutanée.

L'acide rétinoïque possède en effet une très faible solubilité en milieu aqueux en raison de sa forte lipophilie et une mauvaise stabilité en raison de sa sensibilité à l'oxydation et à la lumière.

Dans le brevet US n°4.371.673, il a été proposé d'augmenter la solubilité de l'acide rétinoïque en milieu aqueux par formation de complexes à l'aide de cyclodextrines ou leurs dérivés. Cependant, l'utilisation de la β-cyclodextrine à 1,7% en solution tamponnée pH 7,5 comme agent complexant, nécessite des temps de complexation de plusieurs jours et ne permet pas, par ailleurs, de solubiliser des quantités importantes d'acide rétinoïque. Les études qui ont été réalisées en fonction des enseignements de ce brevet ont en effet mis en évidence que les quantités solubilisées étaient limitées à 0,02%.

Par ailleurs, selon les proportions d'acide rétinoïque à solubiliser, la stabilité physique du complexe est difficile à maitriser et le complexe a tendance à reprécipiter dans le temps sous forme microcristalline.

Dans le brevet US n° 4.727.064, il a été proposé d'augmenter la solubilité de l'acide rétinoïque en milieu aqueux par formation de complexes à l'aide de dérivés de cyclodextrines, en particulier l'hydroxypropyl-β-cyclodextrine (HPβC). Cependant, dans ce brevet la concentration préférée en HPβC est de 40 à 60 % en poids de la composition. Les études qui ont été réalisées en fonction des compositions décrites dans ce brevet mettent en évidence que des gels contenant une telle concentration élevée en HPβC sont irritants au niveau de la peau. Par ailleurs, la réalisation d'un gel à base de polymère de l'acide acrylique est difficilement envisageable à cette concentration d'HPβC.

La présente invention se propose de fournir une nouvelle forme d'administration de l'acide rétinoïque sous forme d'un gel aqueux dans lequel l'acide rétinoïque est plus soluble et stable. Ce gel est par ailleurs agréable à utiliser et présente une bonne tolérance par application locale sur la peau.

L'utilisation d'une composition à usage thérapeutique ou cosmétique sous forme d'un gel présente l'avantage d'être mieux acceptée par les utilisateurs que la forme liquide en raison d'une plus grande facilité d'application sur les parties de la peau à traiter.

Il a maintenant été constaté de manière tout à fait inattendue qu'en diminuant la concentration de HPβC par rapport au taux préférentiel de l'US 4.727.064, jusqu'à supprimer tout effet irritant, il y avait une relation inverse entre la concentration d'HPβC et le pH nécessaire à la solubilisation d'une concentration désirée en acide rétinoïque dans des conditions stables et avec des temps d'agitation transposables industriellement (normalement inférieurs à 24 h).

Les études ont prouvé que les gammes optimales de concentration en HPβC et de pH sont très étroites.

La présente invention a donc pour objet, à titre de produit industriel nouveau, un gel aqueux à base d'acide rétinoïque all-trans ou 13-cis, pour son utilisation dans le domaine pharmaceutique, notamment dermatologique, et dans le domaine cosmétique, dans lequel l'acide rétinoïque est présent à l'état soluble et stable en une proportion de 0,02 à 0,05 % en poids, en présence de 6 à 15 % en poids, et de préférence de 8 à 12 %, d'hydroxypropyl-β-cyclodextrine et de la triéthanolamine en une proportion telle que le pH dudit gel soit compris entre environ 6,8 et 7,4, de préférence entre environ 6,9 et 7,2 et d'un agent gélifiant.

Par l'expression "gel aqueux" telle qu'utilisée selon l'invention on doit généralement entendre un gel ayant un pourcentage en eau supérieur ou égal à 85 % en poids par rapport au poids total du gel.

Selon l'invention, l'augmentation de la solubilité et la meilleure stabilité de l'acide rétinoïque sont obtenues par la présence simultanée de l'hydroxypropyl-β-cyclodextrine et de la triéthanolamine qui permet, entre autre, d'assurer la salification de l'acide rétinoïque, mais qui, utilisée sans la présente d'HPβC, donnerait des solutions très instables.

Il a été remarqué que le choix de la base utilisée influence la qualité de la complexation et la stabilité du gel. La triéthanolamine (TEA) permet d'obtenir une concentration en HPβC et un pH minima. D'autres bases telles que la soude ou la monoéthanolamine (MEA) conduisent par exemple, dans les mêmes conditions à une précipitation du gel dans le temps.

De façon préférentielle, le rapport en poids de l'acide rétinoïque à l'hydroxypropyl-β-cyclodextrine doit être compris entre 0,0015 et 0,0080.

L'agent gélifiant permettant d'obtenir la viscosité voulue est choisi préférentiellement dans la famille des polymères dérivés de l'acide acrylique par exemple le "Carbopol® 940" vendu par la Société GOODRICH.
De préférence la concentration en agent gélifiant est comprise entre 0,1 et 5 % en poids par rapport au poids total de la composition.

La concentration est choisie telle que le gel ait une viscosité comprise de préférence entre environ 1 Pa.s et 60 Pa.s.

Selon une forme de réalisation préférée de l'invention, le gel aqueux contient en outre au moins un agent anti-oxydant, permettant d'augmenter encore la stabilité dans le temps à température ambiante (supérieure à un an) ou à des températures élevées (supérieures ou égales à 45°C).

Parmi ces agents anti-oxydants généralement présents dans le gel en une proportion en poids de 0,001 à 0,3%, on peut notamment mentionner le butylhydroxyanisole (BHA), le sulfite de sodium, l'acide ascorbique ou l'ascorbate de sodium éventuellement associé à un agent complexant des métaux tels que l'acide éthylènediaminetétracétique (EDTA).

Le gel selon l'invention peut également contenir au moins un agent conservateur tel que par exemple le parahydroxy-benzoate de méthyle ou son sel sodique en une proportion en poids comprise entre 0,001 et 0,3%.

Il convient bien entendu que l'agent anti-oxydant et/ou l'agent conservateur soit choisi parmi ceux présentant une bonne stabilité au pH du gel aqueux.

Les gels selon l'invention sont obtenus de préférence en préparant tout d'abord une solution concentrée d'acide rétinoïque dans un mélange aqueux de HPβC et de la TEA choisie dans les proportions requises, puis en préparant séparément à un pH compris entre 6,5 et 7,0 un gel-mère aqueux contenant l'agent gélifiant et éventuellement le ou les agent(s) conservateur(s) et le ou les antioxydant(s).

On procède ensuite au mélange, sous agitation, des quantités adéquates de gel-mère et de solution concentrée d'acide rétinoïque pour obtenir un gel final ayant le taux voulu d'acide rétinoïque.

Ce mode préparatoire qui consiste à réaliser la gélification séparément permet d'assurer la formation du complexe voulu sans le soumettre à une variation de pH due à l'introduction d'un agent gélifiant sous forme acide et sa neutralisation ultérieure.

Le gel aqueux selon l'invention est destiné:
1) au traitement des affections dermatologiques liées à un désordre de la kératinisation portant sur la différenciation et sur la prolifération, notamment pour traiter les acnés vulgaires, comédoniennes, polymorphes, les acnés nodulo kystiques, conglobata, les acnés séniles, les acnés secondaires telles que l'acné solaire, médicamenteuse, professionnelle ;
2) au traitement d'autres types de troubles de la kératinisation, notamment les ichtyoses, les états ichtyosiformes, la maladie de Darier, les kératodermies palmoplantaires, les leucoplasies et les états leucoplasiformes, le lichen ;
3) au traitement d'autres affections dermatologiques liées à un trouble de la kératinisation avec une composante inflammatoire et/ou immunoallergique et, notamment, toutes les formes de psoriasis qu'il soit cutané, muqueux ou unguéal ; ces compositions peuvent également être utilisées dans certaines affections inflammatoires ne présentant pas de trouble de la kératinisation ;
4) au traitement de toutes les proliférations dermiques ou épidermiques qu'elles soient bénignes ou malignes, qu'elles soient d'origine virale telles que verrues vulgaires, les verrues planes et l'épidermodysplasie verruciforme, les proliférations pouvant également être induites par les ultra-violets notamment dans le cadre des épithélioma baso et spino cellulaires ;
5) au traitement d'autres désordres dermatologiques tels que les dermatoses bulleuses et les maladies du collagène ;
6) au traitement de certains troubles ophtalmologiques, notamment les cornéopathies ;
7) pour lutter contre le vieillissement actinique de la peau ou pour réduire les pigmentations ou kératoses actiniques ;
8) pour prévenir ou guérir les stigmates de l'atrophie épidermique et/ou dermique induite par les corticostéroïdes locaux ou systémiques, ou toute autre forme d'atrophie cutanée ;
9) pour favoriser la cicatrisation.

La posologie est variable dans la mesure où elle dépend des affections à traiter et de la concentration, en acide rétinoïque, du gel aqueux.

Le traitement consiste à appliquer au moins une fois par jour, de préférence le soir, la quantité prescrite sur les lésions à traiter. La durée du traitement d'attaque est généralement comprise entre 2 et 15 semaines, et doit être poursuivi par un traitement d'entretien par exemple par des applications deux ou trois fois par semaine.

Le gel aqueux selon l'invention peut également être utilisé dans le domaine cosmétique, en particulier dans l'hygiène corporelle et capillaire.

On va maintenant donner à titre d'illustration plusieurs exemples de gels aqueux selon l'invention.

### EXEMPLES DE GELS

### EXEMPLE 1

On obtient un gel selon l'invention en procédant séparément à la préparation d'un gel mère et d'une solution aqueuse d'acide rétinoïque :

| 1. Gel mère | |
|---|---|
| Polymère de l'acide acrylique vendu par la Société Goodrich sous la dénomination de "Carbopol® 940" | 1,000 g |
| Triéthanolamine à 10 % dans qsp pH = 6,5 | |
| BHA | 0,005 g |
| Parahydroxybenzoate de méthyle | 0,200 g |
| Eau qsp | 100,000 g |

| 2. Solution aqueuse d'acide rétinoïque | |
|---|---|
| Acide rétinoïque all-trans ou 13-cis | 0,050 g |
| HPβC | 20,000 g |
| Triéthanolamine | 0,126 g |
| Eau qsp | 100,000 g |

On procède ensuite au mélange du gel et de la solution aqueuse dans un rapport 50/50 et on obtient un gel ayant un pH de 7,00 et une viscosité de 10 Pa.s.

### EXEMPLE 2

On obtient un gel selon l'invention en procédant séparément à la préparation d'un gel mère et d'une solution aqueuse d'acide rétinoïque :

| 1. Gel mère | |
|---|---|
| Polymère de l'acide acrylique vendu par la Société Goodrich sous la dénomination de "Carbopol® 940" | 1,000 g |
| Triéthanolamine à 10 % dans eau qsp pH 6,7 | |
| BHA | 0,005 g |
| Parahydroxybenzoate de méthyle | 0,200 g |
| Eau qsp | 100,000 g |

| 2. Solution aqueuse d'acide rétinoïque | |
|---|---|
| Acide rétinoïque all-trans ou 13-cis | 0,080 g |
| HPβC | 24,000 g |
| Triéthanolamine | 0,200 g |
| Eau qsp | 100,000 g |

Or procède ensuite au mélange du gel et de la solution aqueuse dans un rapport 50/50 et on obtient un gel ayant un pH de 7,1 et une viscosité de 10 Pa.s.

### EXEMPLE 3

On obtient un gel selon l'invention en procédant séparément à la préparation d'un gel mère et d'une solution aqueuse d'acide rétinoïque :

| 1. Gel mère | |
|---|---|
| Polymère de l'acide acrylique vendu par la Société Goodrich sous la dénomination de "Carbopol® 940" | 1,000 g |
| Triéthanolamine à 10 % dans eau qsp pH = 6,6 | |
| BHA | 0,005 g |
| Parahydroxybenzoate de méthyle | 0,200 g |
| Eau qsp | 100,000 g |

| 2. Solution aqueuse d'acide rétinoïque | |
|---|---|
| Acide rétinoïque all-trans ou 13-cis | 0,040 g |
| HPβC | 16,000 g |
| Triéthanolamine | 0,100 g |
| Eau qsp | 100,000 g |

On procède ensuite au mélange du gel et de la solution aqueuse dans un rapport 50/50 et on obtient un gel ayant un pH de 7,00 et une viscosité de 10 Pa.s.

## Revendications (Revendications pour l'(les) Etat(s) contractant(s) suivant(s): AT, BE, CH, DE, DK, FR, GB, IT, LI, NL, SE)

1. Gel aqueux à base d'acide rétinoïque all-trans ou 13-cis pour une utilisation dans les domaines pharmaceutique et cosmétique, caractérisé par le fait que l'acide rétinoïque est présent, à l'état soluble et stable, en une proportion de 0,02 à 0,05 % en poids, en présence de 6 à 15 % en poids d'hydroxypropyl-β-cyclodextrine et de la triéthanolamine en une proportion telle que le pH dudit gel soit compris entre environ 6,8 et 7,4, et d'un agent gélifiant.

2. Gel selon la revendication 1 caractérisé par le fait qu'il contient un pourcentage en eau supérieur ou égal à 85 % en poids par rapport au poids total du gel.

3. Gel selon l'une des revendications 1 ou 2 caractérisé par le fait que l'hydroxypropyl-β-cyclodextrine est de préférence présente en une proportion comprise entre 8 et 12 % en poids par rapport au poids total du gel.

4. Gel selon l'une quelconque des revendications 1 à 3, caractérisé par le fait que le rapport en poids de l'acide rétinoïque à l'hydroxypropyl-β-cyclodextrine est compris entre 0,0015 et 0,0080.

5. Gel selon l'une quelconque des revendications 1 à 4, caractérisé par le fait que son pH est compris entre 6,9 et 7,2.

6. Gel selon l'une quelconque des revendications 1 à 5, caractérisé par le fait que le gel a une viscosité comprise entre environ 1 Pa.s et 60 Pa.s.

7. Gel selon l'une quelconque des revendications précédentes, caractérisé par le fait que l'agent gélifiant est un polymère dérivé de l'acide acrylique et est présent en une proportion comprise entre 0,1 et 5% en poids.

8. Gel selon l'une quelconque des revendications 1 à 7, caractérisé par le fait qu'il contient en outre un agent anti-oxydant en une proportion en poids de 0,001 à 0,3%.

9. Gel selon la revendication 8, caractérisé par le fait que l'agent anti-oxydant est le butylhydroxyanisole, le sulfite de sodium, l'acide ascorbique ou l'ascorbate de sodium éventuellement associé à un agent complexant des métaux.

10. Gel selon l'une quelconque des revendications 1 à 9, caractérisé par le fait qu'il contient en outre un agent conservateur en une proportion en poids de 0,001 à 0,3%.

11. Gel selon la revendication 10, caractérisé par le fait que l'agent conservateur est le parahydroxybenzoate de méthyle ou son sel sodique.

## Revendications (Revendications pour l'(les) Etat(s) contractant(s) suivant(s): ES, GR)

1. Gel aqueux cosmétique à base d'acide rétinoïque all-trans ou 13-cis, caractérisé par le fait que l'acide rétinoïque est présent, à l'état soluble et stable, en une proportion de 0,02 à 0,05 % en poids, en présence de 6 à 15 % en poids d'hydroxypropyl-β-cyclodextrine et de la triéthanolamine en une proprtion telle que le pH dudit gel soit compris entre environ 6,8 et 7,4, et d'un agent gélifiant.

2. Gel selon la revendication 1 caractérisé par le fait qu'il contient un pourcentage en eau supérieur ou égal à 85 % en poids par rapport au poids total du gel.

3. Gel selon l'une des revendications 1 ou 2 caractérisé par le fait que l'hydroxypropyl-β-cyclodextrine est de préférence présente en une proportion comprise entre 8 et 12 % en poids par rapport au poids total du gel.

4. Gel selon l'une quelconque des revendications 1 à 3, caractérisé par le fait que le rapport en poids de l'acide rétinoïque à l'hydroxypropyl-β-cyclodextrine est compris entre 0,0015 et 0,0080.

5. Gel selon l'une quelconque des revendications 1 à 4, caractérisé par le fait que son pH est compris entre 6,9 et 7,2.

6. Gel selon l'une quelconque des revendications 1 à 5, caractérisé par le fait que le gel a une viscosité comprise entre environ 1 Pa.s et 60 Pa.s.

7. Gel selon l'une quelconque des revendications précédentes, caractérisé par le fait que l'agent gélifiant est un polymère dérivé de l'acide acrylique et est présent en une proportion comprise entre 0,1 et 5% en poids.

8. Gel selon l'une quelconque des revendications 1 à 7, caractérisé par le fait qu'il contient en outre un agent anti-oxydant en une proportion en poids de 0,001 à 0,3%.

9. Gel selon la revendication 8, caractérisé par le fait que l'agent anti-oxydant est le butylhydroxyanisole, le sulfite de sodium, l'acide ascorbique ou l'ascorbate de sodium éventuellement associé à un agent complexant des métaux.

10. Gel selon l'une quelconque des revendications 1 à 9, caractérisé par le fait qu'il contient en outre un agent conservateur en une proportion en poids de 0,001 à 0,3%.

11. Gel selon la revendication 10, caractérisé par le fait que l'agent conservateur est le parahydroxybenzoate de méthyle ou son sel sodique.

## Claims (Claims for the following Contracting State(s): AT, BE, CH, DE, DK, FR, GB, IT, LI, NL, SE)

1. Aqueous gel based on all-trans- or 13-cis-retinoic acid for use in the pharmaceutical and cosmetic fields, characterized by the fact that the retinoic acid is present, in a soluble and stable state, in a proportion of 0.02 to 0.05 % by weight, in the presence of 6 to 15 % by weight of hydroxypropyl-β-cyclodextrin and triethanolamine in a proportion such that the pH of the said gel is between about 6.8 and 7.4, and a gelling agent.

2. Gel according to Claim 1, characterized by the fact that it contains a percentage of water greater than or equal to 85 % by weight relative to the total weight of the gel.

3. Gel according to one of Claims 1 or 2, characterized by the fact that the hydroxypropyl-β-cyclodextrin is preferably present in a proportion of between 8 and 12 % by weight relative to the total weight of the gel.

4. Gel according to any one of Claims 1 to 3, characterized by the fact that the weight ratio of the retinoic acid to the hydroxypropyl-β-cyclodextrin is between 0.0015 and 0.0080.

5. Gel according to any one of Claims 1 to 4, characterized by the fact that its pH is between 6.9 and 7.2.

6. Gel according to any one of Claims 1 to 5, characterized by the fact that the gel has a viscosity of between about 1 Pa.s and 60 Pa.s.

7. Gel according to any one of the preceding claims, characterized by the fact that the gelling agent is a polymer derived from acrylic acid and is present in a proportion of between 0.1 and 5 % by weight.

8. Gel according to any one of Claims 1 to 7, characterized by the fact that it contains, in addition, an antioxidant in a proportion by weight of 0.001 to 0.3 %.

9. Gel according to Claim 8, characterized by the fact that the antioxidant is butylated hydroxyanisole, sodium sulphite, ascorbic acid or sodium ascorbate, optionally combined with a metal-complexing agent.

10. Gel according to any one of Claims 1 to 9, characterized by the fact that it contains, in addition, a preservative in a proportion by weight of 0.001 to 0.3 %.

11. Gel according to Claim 10, characterized by the fact that the preservative is methyl parahydroxybenzoate or its sodium salt.

## Claims (Claims for the following Contracting State(s): ES, GR)

1. Cosmetic aqueous gel based on all-trans- or 13-cis-retinoic acid, characterized by the fact that the retinoic acid is present, in a soluble and stable state, in a proportion of 0.02 to 0.05 % by weight, in the presence of 6 to 15 % by weight of hydroxypropyl-β-cyclodextrin and triethanolamine in a proportion such that the pH of the said gel is between about 6.8 and 7.4, and a gelling agent.

2. Gel according to Claim 1, characterized by the fact that it contains a percentage of water greater than or equal to 85 % by weight relative to the total weight of the gel.

3. Gel according to one of Claims 1 or 2, characterized by the fact that the hydroxypropyl-β-cyclodextrin is preferably present in a proportion of between 8 and 12 % by weight relative to the total weight of the gel.

4. Gel according to any one of Claims 1 to 3, characterized by the fact that the weight ratio of the retinoic acid to the hydroxypropyl-β-cyclodextrin is between 0.0015 and 0.0080.

5. Gel according to any one of Claims 1 to 4, characterized by the fact that its pH is between 6.9 and 7.2.

6. Gel according to any one of Claims 1 to 5, characterized by the fact that the gel has a viscosity of between about 1 Pa.s and 60 Pa.s.

7. Gel according to any one of the preceding claims, characterized by the fact that the gelling agent is a polymer derived from acrylic acid and is present in a proportion of between 0.1 and 5 % by weight.

8. Gel according to any one of Claims 1 to 7, characterized by the fact that it contains, in addition, an antioxidant in a proportion by weight of 0.001 to 0.3 %.

9. Gel according to Claim 8, characterized by the fact that the antioxidant is butylated hydroxyanisole, sodium sulphite, ascorbic acid or sodium ascorbate, optionally combined with a metal-complexing agent.

10. Gel according to any one of Claims 1 to 9, characterized by the fact that it contains, in addition, a preservative in a proportion by weight of 0.001 to 0.3 %.

11. Gel according to Claim 10, characterized by the fact that the preservative is methyl parahydroxybenzoate or its sodium salt.

## Patentansprüche (Patentansprüche für folgende(n) Vertragsstaat(en): AT, BE, CH, DE, DK, FR, GB, IT, LI, NL, SE)

1. Wäßriges Gel auf Basis einer all-trans- oder 13-cis-Retinsäure zur Verwendung auf pharmazeutischem oder kosmetischem Gebiet, wobei das Gel dadurch gekennzeichnet ist, daß die Retinsäure in löslichem und stabilem Zustand in einer Menge von 0,02 bis 0,05 Gew.-% in Anwesenheit von Hydroxypropyl-β-cyclodextrin in einer Menge von 6 bis 15 Gew.-% und von Triethanolamin in einer solchen Menge, daß der pH des Gels zwischen etwa 6,8 und 7,4 liegt, sowie in Anwesenheit eines Getierungsmittels vorliegt.

2. Gel gemäß Anspruch 1, dadurch gekennzeichnet, daß es einen Prozentsatz an Wasser von 85 Gew.-% oder darüber, bezogen auf das Gesamtgewicht des Gels, enthält.

3. Gel gemäß Anspruch 1 oder 2, dadurch gekennzeichnet, daß das Hydroxypropy-β-cyclodextrin vorzugsweise in einer Menge von 8 bis 12 Gew.-%, bezogen auf das Gesamtgewicht des Gels, vorhanden ist.

4. Gel gemäß einem der Ansprüche 1 bis 3, dadurch gekennzeichnet, daß das Gewichtsverhältnis der Retinsäure zu Hydroxypropyl-β-cyclodextrin einen Bereich zwischen 0,0015 und 0,0080 umfaßt.

5. Gel gemäß einem der Ansprüche 1 bis 4, dadurch gekennzeichnet, daß dessen pH einen Bereich zwischen 6,9 und 7,2 umfaßt.

6. Gel gemäß einem der Ansprüche 1 bis 5, dadurch gekennzeichnet, daß das Gel eine Viskosität im Bereich zwischen etwa 1 Pa·s und 60 Pa·s aufweist.

7. Gel gemäß einem der vorstehenden Ansprüche, dadurch gekennzeichnet, daß das Gelierungsmittel ein von Acrylsäure abgeleitetes Polymer darstellt und in einer Menge zwischen 0,1 bis 5 Gew.-% vorliegt.

8. Gel gemäß einem der Ansprüche 1 bis 7, dadurch gekennzeichnet, daß es zusätzlich ein Antioxidans in einer Menge von 0,001 bis 0,3 Gew.-% enthält.

9. Gel gemäß Anspruch 8, dadurch gekennzeichnet, daß das Antioxidans Butylhydroxyanisol, Natriumsulfit, Ascorbinsäure oder Natriumascorbat ist, gegebenenfalls in Verbindung mit einem Metallkomplexbildner.

10. Gel gemäß einem der Ansprüche 1 bis 9, dadurch gekennzeichnet, daß es zusätzlich ein Konservierungsmittel in einer Menge von 0,001 bis 0,3 Gew.-% enthält.

11. Gel gemäß Anspruch 10, dadurch gekennzeichnet, daß das Konservierungsmittel Parahydroxybenzoesäuremethylester oder das Natriumsalz der entsprechenden Säure ist.

## Patentansprüche (Patentansprüche für folgende(n) Vertragsstaat(en): ES, GR)

1. Wäßriges Kosmetikgel auf Basis einer all-trans- oder 13-cis-Retinsäure, dadurch gekennzeichnet, daß die Retinsäure in löslichem und stabilem Zustand in einer Menge von 0,02 bis 0,05 Gew.-% in Anwesenheit von Hydroxypropyl-β-cyclodextrin in einer Menge von 6 bis 15 Gew.-% und von Triethanolamin in einer solchen Menge, daß der pH des Gels zwischen etwa 6,8 und 7,4 liegt, sowie in Anwesenheit eines Gelierungsmittels vorliegt.

2. Gel gemäß Anspruch 1, dadurch gekennzeichnet, daß es einen Prozentsatz an Wasser von 85 Gew.-% oder darüber, bezogen auf das Gesamtgewicht des Gels, enthält.

3. Gel gemäß Anspruch 1 oder 2, dadurch gekennzeichnet, daß das Hydroxypropyl-β-cyclodextrin vorzugsweise in einer Menge von 8 bis 12 Gew.-%, bezogen auf das Gesamtgewicht des Gels, vorhanden ist.

4. Gel gemäß einem der Ansprüche 1 bis 3, dadurch gekennzeichnet, daß das Gewichtsverhältnis der Retinsäure zu Hydroxypropyl-β-cyclodextrin einen Bereich zwischen 0,0015 und 0,0080 umfaßt.

5. Gel gemäß einem der Ansprüche 1 bis 4, dadurch gekennzeichnet, daß dessen pH einen Bereich zwischen 6,9 und 7,2 umfaßt.

6. Gel gemäß einem der Ansprüche 1 bis 5, dadurch gekennzeichnet, daß das Gel eine Viskosität im Bereich zwischen etwa 1 Pa·s und 60 Pa·s aufweist.

7. Gel gemäß einem der vorstehenden Ansprüche, dadurch gekennzeichnet, daß das Gelierungsmittel ein von Acrylsäure abgeleitetes Polymer darstellt und in einer Menge von 0,1 bis 5 Gew.-% vorliegt.

8. Gel gemäß einem der Ansprüche 1 bis 7, dadurch gekennzeichnet, daß es zusätzlich ein Antioxidans in einer Menge von 0,001 bis 0,3 Gew.-% umfaßt.

9. Gel gemäß Anspruch 8, dadurch gekennzeichnet, daß das Antioxidans Butylhydroxyanisol, Natriumsulfit, Ascorbinsäure oder Natriumascorbat ist, gegebenenfalls in Verbindung mit einem Metallkomplexbildner.

10. Gel gemäß einem der Ansprüche 1 bis 9, dadurch gekennzeichnet, daß es zusätzlich ein Konservierungsmittel in einer Menge von 0,001 bis 0,3 Gew.-% enthält.

11. Gel gemäß Anspruch 10, dadurch gekennzeichnet, daß das Konservierungsmittel Parahydroxybenzoesäuremethylester oder das Natriumsalz der entsprechenden Säure ist.
